Europäisches Patentamt

(19) European Patent Office

Office européen des brevets

(11) Publication number : **0 640 341 A1**

(12) # EUROPEAN PATENT APPLICATION

(21) Application number : **94306360.2**

(22) Date of filing : **30.08.94**

(51) Int. Cl.⁶ : **A61K 31/505, A61K 9/50**

(30) Priority : **27.08.93 JP 212458/93**

(43) Date of publication of application :
**01.03.95 Bulletin 95/09**

(84) Designated Contracting States :
**DE FR GB**

(71) Applicant : **MITSUI TOATSU CHEMICALS, INCORPORATED**
**16th Kowa Bldg.,**
**No. 9-20 Akasaka 1-chome**
**Minato-ku, Tokyo (JP)**

(72) Inventor : **Hyugaji, Teruo**
**3-14-14, Shodo,**
**Sakae-Ku**
**Yokohama-shi, Kanagawa-ken (JP)**

Inventor : **Inage, Ikuo**
**1-103, takinosawa,**
**825-1, Endou**
**Fujisawa-shi, Kanagawa-ken (JP)**
Inventor : **Amano, Masaki**
**3-42-7-239, Hirado,**
**Totsuka-ku**
**Yokohama-shi, Kanagawa-ken (JP)**
Inventor : **Sasaki, Masako**
**1-2-22-201, Yuigahama**
**Kamakura-shi, Kanagawa-ken (JP)**
Inventor : **Iizuka, Hajime**
**2142-740-33, Togo**
**Mobara-shi, Chiba-ken (JP)**
Inventor : **Kobayashi, Tadashi**
**2-7-11-202, Kita-yotsui**
**Fukui-shi, Fukui-ken (JP)**

(74) Representative : **Stuart, Ian Alexander et al**
**MEWBURN ELLIS**
**York House**
**23 Kingsway**
**London WC2B 6HP (GB)**

(54) Sustained release pharmaceutical composition and process for producing same.

(57) A sustained release pharmaceutical composition for oral administration having a core comprising (a) 1,3-dimethyl-6-{2-{N-(2-hydroxyethyl)-N-[3-(4-nitrophenyl) propyl]amino}ethylamino}-2,4-(1H,3H)-pyrimidinedione or its acid adduct as an active component, (b) an organic acid, and (c) a water soluble polymer such as polyvinyl alcohol, and a coat comprising a mixture of (d) a water insoluble polymer and (e) a water soluble polymer, the components (a) to (c) being permeable together through the coat so that the active component (a) may be stably dissolved in an intestinal liquid and may not precipitate in a short period of time.

The present invention relates to a sustained release pharmaceutical composition, and more specifically, it relates to a sustained release pharmaceutical composition for oral administration. More concretely, it relates to a sustained release pharmaceutical composition for oral administration of a 1,3-dimethyl-6-{2-{N-(2-hydroxyethyl)-N-[3-(4-nitrophenyl)propyl]amino}ethylamino}-2,4-(1H,3H)-pyrimidinedione acid compound (hereinafter referred to as "the pyrimidinedione derivative" or simply as "the drug") which is a compound useful as a class III type antiarrhythmic drug and which is Compound 8 mentioned in Japanese Patent Application Laid-open No. 173873/1991, and its preparation process.

A pyrimidinedione derivative has been utilized as a class III type antiarrhythmic drug, and it exerts an extremely excellent effect for an arrhythmic remedy which requires a long-term administration of the drug. In addition, the pyrimidinedione derivative is also a drug effective to relieve a heart functional disorder such as heart failure. As a long-term administration manner, an oral administration has been suggested. For example, in Example 18 of Japanese Patent Application Laid-open No. 173873/1991, the pyrimidinedione derivative is sufficiently mixed with lactose and starch, and the resultant mixture is then filled into capsules to manufacture the desired drug. The present inventors actually carried out an oral administration test for the drug. In consequence, they had found for the first time that the drug was absorbed by the stomach and that its concentration in blood reached a high level within 30 minutes from the administration, but the concentration in blood lowered rapidly when 2 or 3 hours had passed since the administration.

In general, the sustained release pharmaceutical composition has many advantages of, for example, reducing the number of administrations of the drug, decreasing side effects and keeping an effective concentration in blood. Because the advantages are important, various pharmaceutical compositions have been developed. For example, as described in Medical Polymer (Kyoritsu Chemical Library, Kyoritsu Publisher) p. 115-150 and elsewhere, there have been suggested a pharmaceutical composition containing a substance capable of easily crumbling in the stomach or the intestines, a pharmaceutical composition obtained by coating tablets or granules of a drug with a hydrophobic substance, a pharmaceutical composition obtained by coating a drug with a semi-permeable film forming polymer, and a pharmaceutical composition obtained by mixing or combining a water soluble or a water slightly soluble polymer with a drug, or adsorbing the drug by the polymer so as to slowly release the drug. Some of the pharmaceutical compositions have been actually put to practical use.

However, the drugs as active components which are contained in the pharmaceutical compositions are different in solubility and absorption sites in the digestive organs on the basis of their characteristics. Consequently, the same release technique is hardly applicable to two drugs, and it is necessary to develop a pharmaceutical composition suitable for each drug.

For example, the above pyrimidinedione derivative is a basic drug, and has general characteristics that it is easily soluble on an acid side of a pH but very little soluble on a neutral side or an alkaline side. That is to say, in the digestive organs, the pyrimidinedione derivative is easily dissolved and released in the stomach owing to an acid therein, but the dissolution and the release of the pyrimidinedione derivative deteriorate in the intestines because the intestines are a neutral or an alkaline state. Furthermore, the deterioration of the solubility of the pyrimidinedione derivative with the increase of the pH is remarkable. For example, in a Japanese pharmacopoeia test liquid No. 1 (pH = 1.2) which corresponds to the pH in the stomach, the solubility of the pyrimidinedione derivative is 1 g/ml or more, but in a Japanese pharmacopoeia test liquid No. 2 (pH = 6.8) which corresponds to the pH in the intestines, the solubility of the pyrimidinedione derivative is 0.1 mg/ml or less. Hence, by the conventional known means for basic pharmaceutical compositions, it is substantially impossible to obtain a sustained release pharmaceutical composition which has a low solubility in the intestines and which can be effectively absorbed.

The present invention intends to provide a sustained release pharmaceutical composition for oral administration effective for an arrhythmic remedy which requires a long-term administration. More specifically, the present invention intends to provide a novel sustained release pharmaceutical composition containing, as an active component, a pyrimidinedione derivative (Japanese Patent Application Laid-open No. 173873/1991) which is a compound useful as a class III type antiarrhythmic drug by the classification of antiarrhythmic drugs described in E. M. Vaughan Williams ("Advances in Drug Research, Vol. 9" edited by N. J. Simmonds, Academic Press in London; 1974; p 69-101).

The pyrimidinedione derivative has a strong basicity as described above, and so it is readily dissolved in the stomach when orally administered, and it is extremely rapidly absorbed therein. In addition, as found for the first time by the present inventors, the pyrimidinedione derivative is quickly metabolized, and in several hours from the oral administration, its concentration in blood noticeably deteriorates. Thus, in order to maintain the concentration in blood at a constant level, the pyrimidinedione derivative is required to be administered little by little every several hours, and so it has been substantially difficult to utilize the pyrimidinedione derivative as the oral drug. For the purpose of solving these problems, the present inventors have tried to restrain the pyrimidinedione derivative from dissolving in the stomach and to dissolve it at a constant ratio for a long period of time in the intestines by the contrivance of a drug morphology such as the formation of a double coat, and as a result, this try has succeeded to some extent.

However, it has been found that most of the pyrimidinedione derivative dissolved precipitates in an intestinal liquid, and thus the absorption of the drug through an intestinal wall cannot be expected. The present inventors have intensively researched with the intention of finding a technique by which the pyrimidinedione derivative is not dissolved in the stomach and the precipitation of the pyrimidinedione derivative does not occur even in a neutral region.

Preferred embodiments of the present invention may provide a sustained release pharmaceutical composition which can prevent a controlled and released drug from precipitating again in a pH region (a neutral state or a weakly alkaline state) in intestines, increase the body's absorption of the drug, and maintain the effective function for a long period of time; and provide a process for preparing the sustained release pharmaceutical composition.

The present inventors have intensively investigated an active component and additives for forming a core as well as a coating composition for effectively controlling the release of the active component. As a result, the present inventors have found a sustained release pharmaceutical composition for oral administration containing the above pyrimidinedione derivative as the active component, and in consequence, the present invention has now been completed.

That is to say, one aspect of the present invention is directed to a sustained release pharmaceutical composition for oral administration comprising a core and a coat (or layer or film) covering the core;

said core comprising essential components of:

(a) 1,3-dimethyl-6-{2-{N-(2-hydroxyethyl)-N-[3-(4-nitrophenyl)propyl]amino}ethylamino}-2,4-(1H,3H)-pyrimidinedione or its acid adduct as an active component,

(b) an organic acid, and

(c) one or more water soluble polymers selected from the group consisting of polyvinyl alcohol, polyethylene glycol, polyvinyl pyrrolidone, hydroxypropyl cellulose, hydroxypropylmethyl cellulose, carboxymethyl cellulose and hydroxyethyl cellulose,

said core having properties capable of stably dissolving the active component (a) in an intestinal liquid without precipitation;

said coat covering the core comprising a mixture of:

(d) a water insoluble polymer, and

(e) a water soluble polymer,

the water insoluble polymer (d) being mixed with the water soluble polymer (e) in such a ratio that the active component (a), the organic acid (b) and the water soluble polymer (c) can penetrate together through the coat in order for the active component (a) passed through the coat not to rapidly precipitate in the intestinal liquid.

Another aspect of the present invention is directed to a process for preparing a sustained release pharmaceutical composition for oral administration comprising a core and a coat covering the core, said process comprising:

the step of forming said core comprising essential components of:

(a) 1,3-dimethyl-6-{2-{N-(2-hydroxyethyl)-N-[3-(4-nitrophenyl)propyl]amino}ethylamino}-2,4-(1H,3H)-pyrimidinedione or its acid adduct as an active component,

(b) an organic acid, and

(c) one or more water soluble polymers selected from the group consisting of polyvinyl alcohol, polyethylene glycol, polyvinyl pyrrolidone, hydroxypropyl cellulose, hydroxypropylmethyl cellulose, carboxymethyl cellulose and hydroxyethyl cellulose,

said core having properties capable of stably dissolving the active component (a) in an intestinal liquid without precipitation; and

the step of forming said coat covering the core form a mixture obtained by mixing a water insoluble polymer (d) with a water soluble polymer (e) in such a ratio that the active component (a), the organic acid (b) and the water soluble polymer (c) can penetrate together through the coat in order for the active component (a) passed through the coat not to rapidly precipitate in the intestinal liquid.

BRIEF DESCRIPTION OF THE DRAWING

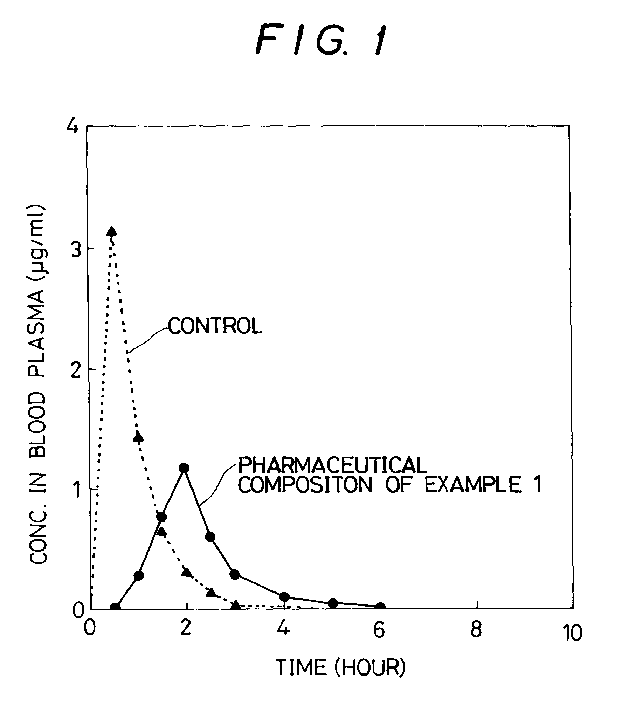

Fig. 1 shows the results of an administration test to a beagle of a sustained release pharmaceutical composition obtained in Example 1.

Next, some preferred embodiments of the present invention will be described in detail.

An active component (a) in a core of the present invention comprises a pyrimidinedione derivative, in particular, Compound 8 (1,3-dimethyl-6-{2-{N-(2-hydroxyethyl) -N-[3-(4-nitrophenyl)propyl]amino}ethylamino}-2,4-(1H,3H) -pyrimidinedione) described in Japanese Patent Application Laid-open No. 173873/1992 and optionally a pharmaceutically acceptable inorganic or organic acid to form an acid adduct (salt).

The optional adduct-forming acid is, for example, an organic acid or an inorganic acid selected from fumaric acid, maleic acid, oxalic acid, malonic acid, succinic acid, tartaric acid, citric acid, lactic acid, benzenesulfonic acid, hydrochloric acid, hydrobromic acid, phosphoric acid, sulfuric acid, nitric acid and methanesulfonic acid.

The core is characterized by comprising this active compound or its acid-added salt (a), an organic acid (b) and a water soluble polymer (c) as essential components. The organic acid (b) has a function for rapidly dissolving the drug substance which is the active component. Examples of the organic acid (b) include acids constituting pharmaceutically acceptable salts mentioned in Japanese Patent Application Laid-open No. 173873/1991, such as adipic acid, maleic acid, citric acid, malonic acid, ascorbic acid, lactic acid, fumaric acid, malic acid, tartaric acid and succinic acid. Preferable are adipic acid, citric acid, ascorbic acid, fumaric acid, malic acid, tartaric acid and succinic acid, and more preferable are fumaric acid, malic acid and succinic acid. The amount of the organic acid (b) to be used is typically in the range of from 0.1 to 200 parts by weight, preferably 20 to 100 parts by weight, based on 100 parts by weight of the drug, i.e., active component (a).

Examples of the water soluble polymer (c) in the core include polyvinyl alcohol, hydroxypropyl cellulose, hydroxypropylmethyl cellulose, polyethylene glycol, carboxymethyl cellulose, polyvinyl pyrrolidone and hydroxyethyl cellulose, and they can be used singly or in combination. Preferable are polyvinyl alcohol and hydroxypropyl cellulose. This water soluble polymer (c) has a function of stably keeping up the dissolving state of the dissolved drug substance as the active component without any precipitation irrespective of a pH. Here, "the dissolving state" does not have to be a strict dissolving state, but it means at least such a dissolving state that the drug substance can be absorbed through an intestinal wall. Incidentally, the typical evaluation of the dissolving state is made on the basis of whether or not the drug substance in the core is stably dissolved in a Japanese pharmacopoeia test liquid No. 2 (pH = 6.8) without any precipitation. The amount of the water soluble polymer (c) to be used is not critical, but it is typically in the range of from 5 to 200 parts by weight, preferably from 5 to 100 parts by weight, more preferably from 10 to 70 parts by weight based on 100 parts by weight of the drug which is the active component (a). If the amount of the water soluble polymer (c) is too little, there cannot be sufficiently exerted the function of the water soluble polymer (c) that the dissolving state is stably maintained in a pH region in an intestinal tube in which the drug substance is mainly absorbed, so that the drug substance inconveniently precipitates with time and the absorption of the drug substance in the intestine noticeably deteriorates. On the other hand, if the amount of the water soluble polymer (c) is too much, the relative amount of the drug substance decreases and so the effect of the drug substance is inconveniently poor, though the dissolving state is stably maintained. Since the water soluble polymer (c) is a polymer, its dissolving rate in water is much lower as compared with the pyrimidinedione derivative. In order to exert the effect of the present invention with the aid of the water soluble polymer (c), it is preferable to achieve a condition under which the soluble polymer begins to dissolve earlier than the drug substance, for example, by that the pyrimidinedione derivative is previously coated with the water soluble polymer (c).

Heretofore, in manufacturing the pharmaceutical composition, a binder is used, and examples of the usable binder include starch, gelatin, gum arabic, cellulose derivatives, polyvinyl pyrrolidone, dextrin and agar. The amount of the binder to be used depends upon its binding power, but in general, it is a small amount of from about 1 to 5 parts by weight. If the binder is used in an amount usable as a usual binder in place of the water soluble polymer (c), the dissolving state of the drug substance cannot be maintained when the pH changes, and in consequence, the drug substance easily precipitates. Furthermore, gum arabic, gelatin, agar and dextrin are water soluble polymers, but even when the binder is used singly in a much more amount than the amount usable as the usual binder, the effect of preventing the drug from precipitating is extremely low. However, they may be used together with the soluble polymer (c) used in the present invention.

In the present invention, the core may contain an excipient, a lubricant, an anticoagulant and a binder which can be used in the technical field of pharmaceutical compositions. Examples of the excipient include sugars such as white sugar and lactose, starch and cellulose, and examples of the lubricant and the anticoagulant include talc, calcium stearate, waxes and silica.

A coat covering the core of the present invention (hereinafter referred to as "the first coat" according to circumstances) is a sustained release coat for giving sustained release properties to the core in an intestinal liquid, and it is characterized by comprising a mixture of a water insoluble polymer (d) and a water soluble polymer (e). The coat can control the release of the active component (a), the organic acid (b) and the water soluble polymer (c) in the core irrespective of the pH. The most important role of this coat is to together or simultaneously release the three components of the pyrimidinedione derivative (a) and the organic acid (b) having a relatively high dissolving rate, and the water soluble polymer (c) having an-extremely low dissolving rate and a large molecular weight, through the coat to the outside of the coat at a controlled release velocity. Incidentally, the typical evaluation of the sustained release coat is made by measuring how the components (a), (b) and (c) in the core are released through the sustained release coat in a Japanese pharmacopoeia test liquid No. 2 (pH = 6.8).

The water insoluble polymer (d) is, for example, one or a combination of two or more selected from the group consisting of ethyl acrylate-methyl methacrylate-trimethylammoniumethyl chloride methacrylate copolymer (which is an acrylic resin commercially available under EUDRAGITE RS from Rohm Pharma GmbH), ethyl acrylate-methyl methacrylate copolymer (which is an acrylic resin commercially available under EUDRAGITE NE from Rohm Pharma GmbH), polymethyl methacrylate, cellulose acetate, cellulose acetate butylate, ethylene-vinyl acetate copolymer, polyvinyl acetate and ethyl cellulose. Preferable are ethyl acrylate-methyl methacrylate-trimethylammoniumethyl chloride methacrylate copolymer, ethyl acrylate-methyl methacrylate copolymer and ethyl cellulose. These polymers are insoluble in water, a stomach liquid and an intestinal liquid but swell and become semi-permeable in water, the gastric liquid (juice) and the intestinal liquid.

The water soluble polymer (e) is, for example, one or a combination of two or more selected from the group consisting of polyvinyl pyrrolidone, hydroxypropyl cellulose, hydroxypropylmethyl cellulose, polyethylene glycol, methyl cellulose, carboxymethyl cellulose, polydimethylaminoethyl methacrylate, hydroxyethyl cellulose and polyvinyl alcohol. Preferable are polyvinyl pyrrolidone, hydroxypropyl cellulose, hydroxypropylmethyl cellulose, polyethylene glycol, hydroxyethyl cellulose and polyvinyl alcohol.

Since the coat is formed from the mixture of the water insoluble polymer (d) and the water soluble polymer (e), the release rate of the core components can be controlled by changing a mixing ratio of both the components and the thickness of the coat. Thus, the mixing ratio of the water insoluble polymer (d) and the water soluble polymer (e) depends upon the composition of the core components to be controlled or the desired release velocity (rate). The water insoluble polymer (d) also forms a semi-permeable coat by itself. However, water and low-molecular weight substances can permeate this semi-permeable coat, but the water soluble polymer (c) which can play the role of a stabilizer important to stably maintain the dissolving state of the drug substance does not permeate the semi-permeable coat. Hence, in the present invention, the coat covering the core is formed from the component system in which the water soluble polymer (e) is dissolved or dispersed in the water insoluble polymer (d). When this coat comes in contact with water, the water soluble polymer (e) alone is dissolved, so that micropores having a size of molecules are formed in the coat, which permits the water soluble polymer (c) used in the core to be released through the coat.

In general, from 1 to 50 parts by weight, preferably from 2 to 30 parts by weight, more preferably from 3 to 20 parts by weight of the water soluble polymer (e) is mixed with 100 parts by weight of the water insoluble polymer (d). The water soluble polymer (e) can be considered to be a certain kind of pores forming agent, and so if the large amount of the water soluble polymer (e) is used, the release velocity of the pyrimidinedione derivative (drug substance) increases excessively, so that the role as the sustained release coat is lost. On the other hand, if the excessively small amount of the water soluble polymer (e) is used, the release of the water soluble polymer (c) which is used as the core component and has the function of stably maintaining the dissolution of the pyrimidinedione derivative is inhibited, so that the pyrimidinedione derivative alone is released through the coat. In consequence, the drug substance cannot be stably maintained in the dissolving state, and it precipitates prior to being absorbed through the intestinal wall, so that the purpose of the coat or coating design cannot be achieved.

The thickness of the coat can be considered to be a coating amount. In general, the coating amount of the coat is preferably in the range of from 5 to 30 parts by weight based on 100 parts by weight of the core.

The coat comprising both the components (d) and (e) as essential components may contain a plasticizer, a lubricant and the like which can be used in the technical field of pharmaceutical compositions, in addition to the above components (d) and (e).

Furthermore, additives for stably forming the coat can be added to the coating components, and examples of the additives include inorganic fine particles such as talc and silica, and organic acid salt fine particles of polyvalent metals such as calcium stearate, magnesium stearate and zinc stearate.

The pharmaceutical composition of the present invention is preferably prepared by two steps. That is to say, the core is first prepared, and the coat for covering the core is then formed.

The core can be prepared by a usual pharmaceutical composition preparation technique. Examples of the applicable preparation technique include a wet extrusion granulation method, a fluidized bed granulation method, an agitation granulation method, a compression method, a spray drying granulation method and a grinding granulation method. A suitable granulation method can be selected from the above granulation methods in consideration of a desired particle diameter, particle distribution and particle surface state, but in view of a particle diameter of 1 to 2 millimeters, the uniform particle diameter distribution, the smooth surface state and the like, the fluidized bed granulation method and the agitation granulation method are most preferable. Additionally, it is an effective means to use a nucleating agent which can usually be used in order to effectively manufacture the dense core particles.

The sustained release coat which covers the core can be formed by a usual coating method. For example, the water insoluble polymer (d) and the water soluble polymer (e) are dissolved or dispersed in a suitable solvent, and the resultant solution is then sprayed on the core components by an air fluidized bed granulation method or a rotary granulation method using a spray, thereby easily forming the sustained release coat around the core particles. Examples of the solvent to be used include water, a lower alcohol, acetone, dichloromethane, ethyl acetate and mixtures thereof. The ratio of the solvent to be used often depends upon the optimum operating conditions of a selected coating device, but it is preferably in the range of from 5 to 25%.

It is preferable in a certain case that the sustained release properties of the pyrimidinedione derivative are further enhanced to inhibit its dissolution in the stomach. It is more preferable for the object of the present invention to impart this effect to the present invention. In this case, the pharmaceutical composition is further coated with a polymer as a second coat (outer coat, hereinafter referred to as "the intestine soluble coat"), and this polymer is dissolved or disintegrated at pH 5 or more which is a pH of a transition area of from the stomach to the intestines. No particular restriction is put on a coating method, so long as it permits the formation of the dense coat, but it is industrially preferable to utilize the coating method for the formation of the first coat (inner coat, the sustained release coat) covering the core. The purpose of the second coat is to retard a sustained release start time of the drug substance. Thus, in compliance with this purpose, the thickness of the second coat can be selected in the range of from 5 to 100 parts by weight based on 100 parts by weight of the core. In the case that the sustained release start time should be set to an early time, the setting can be adjusted by decreasing a coating amount. Incidentally, the typical evaluation of the intestine soluble coat is made on the basis of whether the coat is not substantially dissolved in a Japanese pharmacopoeia test liquid No. 1 (pH = 1.2) and the coat is readily dissolved in a test liquid having a pH 5 or more in the same manner.

Examples of the polymer which is dissolved at pH 5 or more include poly(sodium acrylate), poly(sodium methacrylate), carboxymethyl cellulose, hydroxypropylmethyl cellulose phthalate, carboxymethyl cellulose calcium, cellulose phthalate acetate, carboxylmethylethyl cellulose and hydroxypropylmethyl cellulose acetate succinate.

For the sake of the stabilization of the quality and the workability of the second coat, there can be further used inorganic fine particles such as talc and silica, and organic acid salt fine particles of polyvalent metals such as calcium salts, magnesium salts and zinc salts of organic acids.

As described above, the manufacturing process of the sustained release pharmaceutical composition regarding the present invention comprises, for example, the preparation of the core, the preparation of the first coat covering the core, and the preparation of the second coat further covering the first coat. For example, the drug substance (a) and fumaric acid which is the organic acid (b) are mixed in a V type mixer, and granulation is then carried out by a fluidized bed granulator, while an aqueous polyvinyl alcohol solution that is the water soluble polymer (c) is sprayed. The resultant granules are dried, and then ground by a hammer mill to obtain a fine powder for the core. Next, white sugar is used as a nucleating agent, and the above fine powder is granulated by an agitation granulator, while a mixture of ethanol and water is sprayed, thereby forming spherical granules. Afterward, the coating of the thus formed granules as the cores is carried out by the agitation granulator, while a mixed solution of the water insoluble polymer (d) and the water soluble polymer (e) is sprayed, to form the first coat. Next, the obtained spherical particles are coated with the second coat by the use of the agitation granulator, while a water-ethanol solution of the intestine soluble polymer is sprayed, thereby obtaining the sustained release pharmaceutical composition of the present invention.

The above procedure is merely one example, and the manufacturing means of the present invention are not limited to this procedure.

Now, the present invention will be described in detail with respect to examples. In the undermentioned examples, 1,3-dimethyl-6-{2-{N-(2-hydroxyethyl)-N-[3-(4-nitrophenyl)propyl]amino}ethylamino}-2,4-(1H,3H)-pyrimidinedione·hydrochloride was used as a drug substance. This drug substance is Compound 8 described in Example 4 in Japanese Patent Application Laid-open No. 173873/1991 except that the fumaric acid salt is converted into hydrochloride.

\<Example 1\>

(Preparation of cores)

| Components | |
|---|---|
| White sugar particle (carrier) | 300 g |
| Drug substance | 500 g |
| Lactose | 1,500 g |
| Fumaric acid | 250 g |
| Polyvinyl alcohol | 104 g |

300 g of a white sugar particle (trade name Nonparel, made by Floint Industry Co., Ltd.) of 20 to 24 mesh was agitated by a centrifugal fluid type granulator (CF granulator, made by Floint Industry Co., Ltd.), and a mixture of 500 g of a drug, 1,500 g of lactose and 250 g of fumaric acid was added thereto little by little, while 539 g of a 17.6% aqueous solution of polyvinyl alcohol was sprayed on the carrier being agitated, to form coated granules. After the granulation, the granules were dried at 60°C overnight to obtain 2,620 g of yellow crude granules (average particle diameter = 1.1 mm).

(Preparation of sustained release coating)

| Components | |
|---|---|
| EUDRAGITE RS | 12.6 g |
| Polyvinyl pyrrolidone | 2.4 g |

150 g of the already obtained crude granules was agitated by an agitation fluidized bed granulator (New Marumerizer, made by Fuji Powdal Co., Ltd.). Next, 12.6 g of EUDRAGITE RS (which is an acrylic resin commercially available from Rohm Pharma GmbH) and 2.4 g of polyvinyl pyrrolidone were dissolved in a mixture of 201 g of ethanol and 35 g of water, and the resultant solution was sprayed on the crude granules over about 30 minutes, and then dried by aeration at 60°C to obtain 160 g of the coated granules (average particle diameter = 1.1 mm).

\<Examples 2 to 6\>

Using the yellow crude granules obtained in Example 1 as cores, the same procedure as in Example 1 was carried out except that the components shown in Table 1 were used as components for sustained release coat, thereby obtaining the coated granules.

[Table 1]

| Components of coat | Ratio (%) of components | | | | |
|---|---|---|---|---|---|
| | Ex. 2 | 3 | 4 | 5 | 6 |
| EUDRAGITE RS | 88 | 80 | 90 | 90 | |
| Ethyl cellulose | | | | | 90 |
| Polyvinyl pyrrolidone | 12 | 20 | | | 10 |
| Polyethylene glycol | | | | 10 | |
| Hydroxypropyl cellulose | | | 10 | | |

\*   The ratios of the components in 15 g of the
coat are shown.

<Release Test>

In accordance with a release test method No. 2 (Puddle method) described in the 12th revision of Japanese pharmacopoeia, 1,000 mg (in terms of a drug) of the granules obtained in each of Examples 1 to 6 was added to 900 ml of each of Japanese pharmacopoeia No. 1 liquid and No. 2 liquid. Afterward, the solution was stirred at a temperature of 37°C at a rotational speed of 100 rpm. Every a certain time, 5 ml of the test solution was sampled, and then filtered through a membrane filter of 0.45 μm, and an absorbance at 310 nm was measured. From this absorbance, an amount of the released drug was determined, and a release ratio was then calculated. The results of the release test are shown in Table 2. It is apparent from the result of the Table 2 that the pharmaceutical compositions of the present invention exhibit a controlled release performance and a high dissolution stability even in the test liquid No. 2.

[Table 2]

| Ex. | Test liquid | Release ratio (%) | | | | | | |
|---|---|---|---|---|---|---|---|---|
| | | 30 min. | 1 hr. | 2 hr. | 3 hr. | 5 hr. | 24 hr. | |
| 1 | 2 | 26.7 | 59.0 | 93.8 | 98.5 | 97.9 | 100.3 | |
| | 1 | 23.1 | 51.8 | 84.1 | 99.7 | 100.2 | | |
| 2 | 2 | 3.6 | 15.3 | 30.1 | 43.9 | 70.4 | 99.0 | |
| 3 | 2 | 45.0 | 81.1 | 90.0 | 96.3 | 98.1 | 99.2 | |
| | 1 | 35.9 | 70.5 | 83.3 | 85.9 | 90.0 | 93.5 | |
| 4 | 2 | 3.5 | 14.8 | 30.1 | 41.5 | 68.5 | 95.0 | |
| 5 | 2 | 18.4 | 31.1 | 53.4 | 65.1 | 82.0 | 98.1 | |
| 6 | 2 | 3.5 | 14.2 | 30.3 | 42.1 | 68.7 | 94.8 | |

* The release ratio is a percent by weight of the amount of the release drug to the amount of the used drug.

<Example 7>

2 kg of a drug substance and 1 kg of fumaric acid were mixed for 15 minutes in a V type mixer, and granulation was then carried out by a fluidized bed granulator, while 2.67 kg of an aqueous PVA solution having a concentration of 15% was sprayed. After sufficient drying, the granules were ground by a hammer mill to obtain a powder having an average particle diameter of 21 μm. Next, 500 g of white sugar (trade name Nonparel 103) was used as a nucleating agent to spread 1,895 g of the above powder. While a 50% aqueous ethanol solution was sprayed as a spreading agent, granulation was carried out by the use of an agitation granulator to obtain crude granules having an average particle diameter of 1.3 mm. The composition of the thus obtained crude granules was as follows:

| Components | |
|---|---|
| White sugar | 14.8% |
| Drug substance | 57.9% |
| Fumaric acid | 25.4% |
| PVA | 9.1% |

The thus obtained crude granules was coated with sustained release coat at a ratio of 100 parts of the former to 11.7 parts of the latter by the use of the agitation granulator, while ethanol containing 15% of water was sprayed as a solvent. The composition of the sustained release coat was as follows:

| Components | |
|---|---|
| EUDRAGITE RS | 90 parts |
| PVP | 9.1 parts |
| Magnesium Stearate | 7 parts |
| Triethyl citrate | 10 parts |

The obtained sustained release pharmaceutical composition was further coated with an intestine soluble coat (the second coat) at a ratio of 11 parts of the second coat to 100 parts of the crude granules by the agitation granulator, while an ethanol solution containing 15% of water was sprayed. The composition of the intestine soluble film was as follows:

| Composition | |
|---|---|
| Hydroxypropyl methyl cellulose phthalate | 100 parts |
| Triethyl citrate | 10 parts |

<Comparative Example 1>

300 g of a white sugar particle carrier was used as a nucleating agent, and 1,000 g of a powdery drug was then granulated by an agitation granulator, while water was sprayed, to obtain spherical particles. Next, the particles as cores were coated with the first coat and the second coat to obtain a sustained release pharmaceutical composition having an average particle diameter of 1.2 mm.

<Comparative Example 2>

The same procedure as in Example 7 was carried out except that in the coating of the first sustained release coat, a water soluble polymer (e) was omitted, to prepare a sustained release pharmaceutical composition.

<Comparative Example 3>

The same procedure as in Example 7 was carried out except that in the coating of the first sustained release coat, a mixing ratio of EUDRAGITE (d) and polyvinyl pyrrolidone (e) was 1:1, to prepare a sustained release pharmaceutical composition.

<Examples 8 to 12>

Cores were prepared in the same manner as in Example 7, and these granules were successively coated with the first coat (sustained release coat) and the second coat (intestine soluble coat) by the use of the same agitation fluid granulator (New Marumerizer) as in Example 7. Compositions of the first coat and the second coat are shown in Table 3. By any prescription, yellow spherical granules having an average particle diameter of 1.3 mm could be obtained.

In accordance with the procedure described in Examples 1 to 6, each product of Examples 7 to 12 was subjected to a release test, and the results of the test are shown in Table 4. In consequence, it is apparent that the first coat contributes to the sustained release of the drug, and the second coat is effective to retard a sustained release start time of the drug. Furthermore, Table 5 shows the results of similar release tests for the products of Comparative Examples 1 to 3.

[Table 3]

(Ratio of coat components to 150 g of cores)

| | | Ex. 8 | Ex. 9 | Ex. 10 | Ex. 11 | Ex. 12 |
|---|---|---|---|---|---|---|
| First Coat | EUDRAGITE RS | 13.5 g | 8.0 g | 20.0 g | 13.0 g | 13.5 g |
| | Ethyl cellulose | | | | 1.0 g | 1.0 g |
| | PVP | 1.0 g | 0.5 g | 2.0 g | 1.0 g | |
| | Magnesium stearate | 0.5 g | 0.5 g | 1.0 g | | |
| | Talc | | | | | 2.0 g |
| Second Coat | Hydroxypropyl methylcellulose phthalate | 20.0 g | 25.0 g | 30.0 g | 20.0 g | 20.0 g |
| | Triethyl citrate | 3.0 g | 3.0 g | 3.0 g | 3.0 g | 3.0 g |
| | Zinc stearate | 0.5 g | 1.0 g | | | 0.5 g |
| | Magnesium stearate | | | | 0.5 g | |

[Table 4]

| | Test liquid | Release ratio (%) | | | | | |
|---|---|---|---|---|---|---|---|
| | | 30 min. | 1 hr. | 2 hr. | 3 hr. | 5 hr. | 24 hr. |
| Ex. 7 | 2 | 11.2 | 27.5 | 50.8 | 63.4 | 80.3 | 99.1 |
| | 1 | 0.0 | 0.8 | 0.7 | 1.8 | 2.4 | 48.4 |
| Ex. 8 | 2 | 14.3 | 32.6 | 55.2 | 70.1 | 89.7 | 99.8 |
| | 1 | 1.1 | 0.8 | 2.7 | 4.1 | 8.3 | 65.3 |
| Ex. 9 | 2 | 22.3 | 48.0 | 75.2 | 88.3 | 99.6 | 98.8 |
| | 1 | 0.2 | 0.7 | 1.8 | 3.5 | 6.0 | 67.0 |
| Ex.10 | 2 | 8.3 | 20.0 | 41.8 | 58.7 | 79.3 | 98.1 |
| | 1 | 0.3 | 0.5 | 1.1 | 2.5 | 4.1 | 58.8 |
| Ex.11 | 2 | 11.7 | 23.0 | 46.0 | 63.1 | 84.4 | 99.1 |
| | 1 | 1.2 | 1.5 | 1.8 | 5.3 | 9.7 | 67.5 |
| Ex.12 | 2 | 7.5 | 18.5 | 35.0 | 50.2 | 70.2 | 99.7 |
| | 1 | 0.9 | 1.0 | 1.8 | 6.2 | 11.5 | 62.3 |

[Table 5]

| | Test liquid | Release ratio (%) | | | | | |
|---|---|---|---|---|---|---|---|
| | | 30 min. | 1 hr. | 2 hr. | 3 hr. | 5 hr. | 24 hr. |
| Comp. Ex. 1 | 2 | 10.7 | 12.3 | 12.0 | 12.5 | 11.9 | 11.1 |
| | 1 | 22.8 | 50.5 | 85.1 | 99.5 | 100.1 | |
| Comp. Ex. 2 | 2 | 9.3 | 17.5 | 15.8 | 13.8 | 14.0 | 13.1 |
| | 1 | 0.0 | 1.2 | 0.6 | 2.3 | 2.8 | 38.1 |
| Comp. Ex. 3 | 2 | 87.8 | 100.6 | 99.8 | 100.1 | 99.1 | 99.4 |
| | 1 | 0.0 | 0.8 | 3.9 | 5.8 | 18.0 | 68.3 |

As is apparent from the results of Comparative Example 1, in the case that the organic acid (b) and the water soluble polymer (c) are not contained in the core, it is apparent that the drug does not stably release in the test liquid No. 2.

It is apparent from the results of Comparative Example 2 that in the case that the water soluble polymer (e) is omitted from the first coat (the sustained release coat), pores enough to pass the water soluble polymer (c) therethrough are not formed in the coat, and so the drug alone is passed through the coat in the test liquid No. 2 and it precipitates, with the results that the drug does not stably release.

In Comparative Example 3, when the first coat (the sustained release coat) is formed, the amount of the water soluble polymer (e) is used in excessively large quantities, the excessive pores are formed, so that a release velocity is too high and the release control function as the sustained release film cannot be exerted. Thus, the core components are inconveniently released in a short period of time in the test liquid No. 2.

<Results of an administration test to beagles>

In order to confirm an efficacy of the pharmaceutical composition of the present invention, an administration test to a beagle was carried out to measure a change of its concentration in blood plasma to time. The pharmaceutical composition of Example 1 and a usual pharmaceutical composition as a control having no sustained release properties were each administered to the beagle in an amount of 10 mg/kg. The results are shown in Fig. 1, and the control of a maximum concentration in blood plasma and the retard of a time till the attainment of the maximum concentration were observed, and it was confirmed that the pharmaceutical composition of the present invention was effective.

As is apparent from the above results, the present invention can provide the effect that a pyrimidinedione derivative mentioned in Japanese Patent Application Laid-open No. 173873/1991 can be released in a desired time and a desired concentration in blood can be maintained for a long period of time.

## Claims

1. A sustained release pharmaceutical composition for oral administration comprising a core and a coat covering the core;
   said core comprising as essential components:
   (a) 1,3-dimethyl-6-{2-{N-(2-hydroxyethyl)-N-[3-(4-nitrophenyl)propyl]amino}ethylamino}-2,4-(1H,3H)-pyrimidinedione or its acid adduct as an active component,
   (b) an organic acid, and
   (c) one or more water soluble polymers selected from polyvinyl alcohol, polyethylene glycol, polyvinyl pyrrolidone, hydroxypropyl cellulose, hydroxypropylmethyl cellulose, carboxymethyl cellulose and hydroxyethyl cellulose.
   said core having properties capable of stably dissolving the active component (a) in an intestinal liquid without precipitation;
   said coat covering the core comprising a mixture of:
   (d) a water insoluble polymer, and
   (e) a water soluble polymer,
   the water insoluble polymer (d) being mixed with the water soluble polymer (e) in such a ratio that the active component (a), the organic acid (b) and the water soluble polymer (c) can penetrate together through the coat in order for the active component (a) passed through the coat not to rapidly precipitate in the intestinal liquid.

2. The sustained release pharmaceutical composition according to Claim 1 wherein the amount of the water soluble polymer (c) for forming the core is in the range of from 5 to 200 parts by weight based on 100 parts by weight of the active component (a).

3. The sustained release pharmaceutical composition according to claim 1 or 2 wherein the water insoluble polymer (d) for forming the coat is selected from ethyl acrylate-methyl methacrylate-trimethylammoniumethyl chloride methacrylate copolymer, ethyl acrylate-methyl methacrylate copolymer, polymethyl methacrylate, cellulose acetate, cellulose acetate butylate, ethylene-vinyl acetate copolymer, polyvinyl acetate and ethyl cellulose.

4. The sustained release pharmaceutical composition according to Claim 1, 2 or 3 wherein the water soluble polymer (e) for forming the coat is selected from polyvinyl pyrrolidone, hydroxypropyl cellulose, hydroxypropylmethyl cellulose, polyethylene glycol, hydroxyethyl cellulose, methyl cellulose, polyvinyl alcohol, carboxymethyl cellulose and polydimethylaminoethyl methacrylate.

5. The sustained release pharmaceutical composition according to any preceding claim wherein the organic acid (b) for forming the core is selected from adipic acid, maleic acid, malonic acid, fumaric acid, malic acid, tartaric acid, ascorbic acid, lactic acid, citric acid and succinic acid.

6. The sustained release pharmaceutical composition according to any preceding claim wherein the active component (a) is an adduct with an organic acid or an inorganic acid selected from fumaric acid, maleic acid, oxalic acid, malonic acid, succinic acid, tartaric acid, citric acid, lactic acid, benzenesulfonic acid, hydrochloric acid, hydrobromic acid, phosphoric acid, sulfuric acid, nitric acid and methanesulfonic acid.

7. The sustained release pharmaceutical composition according to any preceding claim further comprising a second coat of a polymer which is dissolved at pH 5 or more.

8. The sustained release pharmaceutical composition according to Claim 7 wherein the polymer which is dissolved at pH5 or more is selected from poly(sodium acrylate), poly(sodium methacrylate), carboxymethyl cellulose, hydroxypropylmethyl cellulose phthalate, carboxymethyl cellulose calcium, cellulose phthalate acetate, carboxylmethylethyl cellulose and hydroxypropylmethyl cellulose acetate succinate.

9. The sustained release pharmaceutical composition according to Claim 1 which is orally administered.

10. A process for preparing a sustained release pharmaceutical composition for oral administration comprising a core and a coat covering the core, said process comprising:
    the step of forming said core comprising as essential components:
    (a) 1,3-dimethyl-6-{2-{N-(2-hydroxyethyl)-N-[3-(4-nitrophenyl)propyl]amino}ethylamino}-2,4-(1H,3H)-pyrimidinedione or its acid adduct as an active component,
    (b) an organic acid, and
    (c) one or more water soluble polymers selected from polyvinyl alcohol, polyethylene glycol, polyvinyl pyrrolidone, hydroxypropyl cellulose, hydroxypropylmethyl cellulose, carboxymethyl cellulose and hydroxyethyl cellulose,
    said core having properties capable of stably dissolving the active component (a) in an intestinal liquid without precipitation; and
    the step of forming said coat covering the core from a mixture obtained by mixing a water insoluble polymer (d) with a water soluble polymer (e) in such a ratio that the active component (a), the organic acid (b) and the water soluble polymer (c) can penetrate together through the coat in order for the active component (a) passed through the coat not to rapidly precipitate in the intestinal liquid.

11. The process for preparing a sustained release pharmaceutical composition according to Claim 10 wherein the amount of the water soluble polymer (c) for forming the core is in the range of from 5 to 200 parts by weight based on 100 parts by weight of the active component (a).

12. The process for preparing a sustained release pharmaceutical composition according to Claim 10 or 11 wherein the water insoluble polymer (d) for forming the coat is selected from ethyl acrylate-methyl methacrylate-trimethylammoniumethyl chloride methacrylate copolymer, ethyl acrylate-methyl methacrylate copolymer, polymethyl methacrylate, cellulose acetate, cellulose acetate butylate, ethylene-vinyl acetate copolymer, polyvinyl acetate and ethyl cellulose.

13. The process for preparing a sustained release pharmaceutical composition according to claim 10, 11 or 12 wherein the water soluble polymer (e) for forming the coat is selected from polyvinyl pyrrolidone, hydroxypropyl cellulose, hydroxypropylmethyl cellulose, polyethylene glycol, hydroxyethyl cellulose, methyl cellulose, polyvinyl alcohol, carboxymethyl cellulose and polydimethylaminoethyl methacrylate.

14. The process for preparing a sustained release pharmaceutical composition according to claim 10, 11, 12 or 13 wherein the organic acid (b) for forming the core is selected from adipic acid, maleic acid, malonic acid, fumaric acid, malic acid, tartaric acid, ascorbic acid, lactic acid, citric acid and succinic acid.

15. The process for preparing a sustained release pharmaceutical composition according to any of claims 10-14 wherein the active component (a) is an adduct with an organic acid or an inorganic acid selected from fumaric acid, maleic acid, oxalic acid, malonic acid, succinic acid, tartaric acid, citric acid, lactic acid, benzenesulfonic acid, hydrochloric acid, hydrobromic acid, phosphoric acid, sulfuric acid, nitric acid and methanesulfonic acid.

16. The process for preparing a sustained release pharmaceutical composition according to any of claims 10-15 further comprising, after the step of forming the coat, a step of forming a second coat by covering with a polymer which is dissolved at pH5 or more.

17. The process for preparing a sustained release pharmaceutical composition according to Claim 16 wherein the polymer which is dissolved at pH 5 or more is selected from poly(sodium acrylate), poly(sodium methacrylate), carboxymethyl cellulose, hydroxypropylmethyl cellulose phthalate, carboxymethyl cellulose calcium, cellulose phthalate acetate, carboxylmethylethyl cellulose and hydroxypropylmethyl cellulose acetate succinate.

18. The process for preparing a sustained release pharmaceutical composition according to any of claims 10-17 which further comprising the step of beforehand coating the active component (a) with the water soluble polymer (c).

# F I G. 1

European Patent Office

# EUROPEAN SEARCH REPORT

Application Number

EP 94 30 6360

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,A | EP-A-0 369 627 (MITSUI TOATSU CHEMICALS INC.) <br> * claims 1,9,17-20 * <br> * example 4 * <br> * page 23, line 6 - line 16 * <br>--- | 1-18 | A61K31/505 <br> A61K9/50 |
| A | EP-A-0 454 498 (MITSUI TOATSU CHEMICALS INC.) <br> * the whole document * <br>--- | 1-18 | |
| A | CHEMICAL ABSTRACTS, vol. 112, no. 8, 19 February 1990, Columbus, Ohio, US; abstract no. 62669, <br> * abstract * <br> & JP-A-01 175 937 (MITSUI TOATSU CHEMICALS INC.) 12 July 1989 <br>----- | 1-18 | |
| | | | TECHNICAL FIELDS SEARCHED (Int.Cl.6) |
| | | | A61K |

The present search report has been drawn up for all claims

| Place of search | Date of completion of the search | Examiner |
|---|---|---|
| THE HAGUE | 29 November 1994 | Scarponi, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)